# EUROPEAN PATENT APPLICATION

(11) **EP 3 608 665 A1**
(43) Date of publication of application: **12.02.2020**
(21) Application number: 17904530.7
(22) Date of filing: 07.04.2017
(51) Int. Cl.: G01N 30/04, G01N 33/532, G01N 33/543

(54) **METHOD FOR MANUFACTURING PURIFIED PRODUCT OF PROTEIN-MODIFIED PHOSPHOR-INTEGRATED PARTICLE, METHOD FOR MANUFACTURING FLUORESCENT STAINING LIQUID, PURIFIED PRODUCT OF PROTEIN-MODIFIED PHOSPHOR-INTEGRATED PARTICLE, AND FILTER FOR PURIFYING FLUORESCENT STAINING LIQUID AND PROTEIN-MODIFIED PHOSPHOR-INTEGRATED PARTICLE**

(71) Applicant: Konica Minolta, Inc., Tokyo 100-7015 (JP)
(72) Inventor: ISODA, Takeshi, Tokyo 100-7015 (JP)
(74) Representative: MFG Patentanwälte Meyer-Wildhagen Meggle-Freund Gerhard PartG mbB
(86) International application number: PCT/JP2017/014568
(87) International publication number: WO 2018/185942

(57) **Abstract**

The present invention relates to a method for manufacturing a purified product of a protein-modified phosphor-integrated particle, a method for manufacturing a fluorescent staining liquid, a purified product of a protein-modified phosphor-integrated particle, and a filter for purifying a fluorescent staining liquid and a protein-modified phosphor-integrated particle, in which the method for manufacturing a purified product of a protein-modified phosphor-integrated particle includes a purification step for separating protein-modified phosphor-integrated particles from an impurity by bringing a solution containing the phosphor-integrated particles (protein-modified phosphor-integrated particles), of which surfaces are modified with a biological substance-binding protein, and the impurity originating from the manufacturing process thereof into contact with a filter having a substance (protein-binding substance) capable of reversibly binding to the biological substance-binding protein supported thereon.

## Description

### Technical Field

The present invention relates to a method for manufacturing a purified product of a protein-modified phosphor-integrated particle, a method for manufacturing a fluorescent staining liquid, a purified product of a protein-modified phosphor-integrated particle, and a filter for purifying a fluorescent staining liquid and a protein-modified phosphor-integrated particle.

### Background Art

Pathological diagnosis is a method in which a sample slide is prepared by using tissues or cells collected from a patient, and based on a stained image obtained by staining according to a predetermined method, by observing morphology of the cells or tissues and evaluating the expression state of a particular biological substance, various situations including whether the patient suffers from a particular disorder or not or a particular therapeutic drug is effective or not are determined.

In pathological diagnosis, a method of diagnosing prognosis of a breast cancer patient or predicting the therapeutic effect of a molecular-targeted therapeutic agent "trastuzumab" (trade name "Herceptin" (registered trademark), anti HER2 monoclonal antibody) by quantification and evaluation of the HER2 gene (HER2/neu, c-erbB-2) as one of cancer genes and/or HER2 protein, which is a membrane protein produced from the HER2 gene, by using a specimen prepared by collecting tumor tissues is widely carried out. The HER2 protein is believed to perform signal transduction by forming a homodimer or a heterodimer bound with activated EGFR (HER1), HER3, or HER4, and it is believed that the HER2 protein functions as a growth factor receptor in cancer cells (until now, no endogenous ligand binding to HER2 has been known).

As a HER2 test method having tumor tissues as a subject, the immunohistochemical (IHC) method by which the HER2 protein is stained and fluorescent in situ hybridization (FISH) method by which the HER2 gene is stained can be mentioned. With regard to the test order and criteria for determination (score), ASCO/CAP HER2 Testing Guidelines (prepared in 2007, and revised in 2013) issued by The American Society of Clinical Oncology and College of American Pathologists is widely used in many countries. In Japan as well, HER2 Testing Guidelines based on revised ASCO/CAP HER2 Testing Guidelines (HER2 Testing Guidelines 4th edition, Breast Cancer HER2 Testing and Pathology Committee, April 2014) is used.

As the IHC method, a method in which an enzyme-labeled antibody is brought into contact with a specimen, and, after having direct or indirect binding between the antibody and a protein as a detection target by using an antigen-antibody reaction, and color development is caused according to a reaction of a substrate corresponding to the enzyme is generally used. For example, a DAB staining method in which peroxidase is used as an enzyme and diaminobenzidine is used as a substrate is widely employed.

However, in the staining method like DAB staining method which is based on a reaction between an enzyme and a substrate, the color intensity is greatly affected by conditions such as temperature or time, and thus there is a problem that accurate estimation of the actual amount of a protein of interest from the color intensity is difficult to achieve.

Therefore, in recent years, for labeling a protein, a method of using nano-sized particles in which plural phosphors such as an organic fluorescent pigment or a semiconductor nano particle (quantum dot) are integrated, i.e., phospher integrated particle (PID, may be also referred to as "fluorescent substance-integrated nano particles" or the like), has been proposed and increasingly put into practice. When a protein of interest is labeled with a phosphor-integrated particle and an excitation light suitable for the phosphor is irradiated thereto, the labeled protein can be observed as a high-luminance bright spot and the position and amount of protein expression can be accurately evaluated. In addition, as the phosphor-integrated particles are less likely to fade compared to a staining agent that has been conventionally used, the method has an advantage that observation or image capturing can be made for a relatively long period of time.

As a method for fluorescent labeling of a protein contained in a specimen by using phosphor-integrated particles, the following methods are known. For example, a fluorescent labeling method (secondary antibody method with combined use of avidin-biotin) is disclosed in the examples of Patent Literature 1 (WO 2014/136885 A) in which a phosphor-integrated particle of which surface is modified with streptavidin (pigment resin particles) is prepared, a primary antibody (anti HER2 rabbit monoclonal antibody) is allowed to bind to an antigen (HER2 protein), and subsequently, after allowing a biotin-labeled secondary antibody (anti rabbit IgG antibody) to bind to the primary antibody, the streptavidin-modified phosphor-integrated particle is allowed to bind to the secondary antibody.

In that case, production of the streptavidin-modified phosphor-integrated particle is carried out according to the following method.
(i) By using a melamine resin as a base body, a resin particle in which fluorescent pigments are integrated (enclosed) is formed, and thereafter, an amino group is introduced to the particle surface by reacting the melamine resin with 3-aminopropylmethoxysilane as a silane coupling agent, and the amino group is further reacted with succinimidyl-[(N-maleimidopropionamido)-dodecaethyleneglycol]ester as a linker to introduce a maleimide group to a surface of the melamine resin particle.
(ii) Streptavidin and N-succinimidyl S-acetylthioacetic acid are reacted with each other to introduce a thiol group to the streptavidin.
(iii) The maleimide group of the melamine resin particle in above (i) and the thiol group of the streptavidin in above (ii) are reacted with each other for their binding.

Furthermore, disclosed in Patent Literature 2 (WO 2016/129444 A) is an antibody-bound phosphor-integrated nano particle in which a suitable number of antibody is bound per unit area of a particle surface according to a reaction between an antibody, in which a thiol group (-SH) is formed by reduction of a suitable number of disulfide bond (-S-S-) based on a treatment using specific reducing agent, and a phosphor-integrated nano particle, in which a binding group capable of reacting with the thiol group (e.g., maleimide group) is introduced to the surface. As the antibody-bound phosphor-integrated nano particle inhibits the binding of plural phosphor-integrated nano particles to one antibody, aggregation or precipitation of the particle during storage can be suppressed. It is believed that the antibody can be either a primary antibody used for a direct method of the IHC method or a secondary antibody or the like used for an indirect method.

For example, in the examples of Patent Literature 2, an embodiment of carrying out immunostaining of HER2 by using an antibody-modified fluorescent pigment-enclosed silica nano particle produced by the following method is disclosed.
(i) By using 3-aminopropyltrimethoxysilane as a raw material and 5-carboxytetramethylrhodamine (registered trademark "TAMRA") as a fluorescent pigment, a fluorescent pigment-enclosed silica nano particle is formed, and, by reacting the silica nano particle with succinimidyl-[(N-maleimidopropionamido)-dodecaethyleneglycol]ester (product name: "SM(PEG)₁₂") as a linker, a maleimide group is introduced to the particle surface.
(ii) A primary antibody (anti HER2 rabbit monoclonal antibody) or a secondary antibody (anti rabbit IgG antibody) is treated with 2-mercaptoethanol or the like as a reducing agent and a disulfide group is reduced to yield a thiol group.
(iii) The maleimide group of the silica nano particle in above (i) and the thiol group of the primary antibody or secondary antibody in above (ii) are reacted with each other for their binding.

In Patent Literature 3 (WO 2015/163209 A), a storage liquid containing buffer, protein, surfactant, or the like to be used for storing an antibody-bound phosphor-integrated particle until the use for tissue staining after manufacture is disclosed, and, by enhancing the storage dispersion stability of an antibody-bound phosphor-integrated nano particle during storage, precipitation and/or aggregation are/is inhibited, and thus the working effect of suppressing a coarse mass of the particle during tissue staining is exhibited.

### Citation List

### Patent Literature

Patent Literature 1: WO 2014/136885 A
Patent Literature 2: WO 2016/129444 A
Patent Literature 3: WO 2015/163209 A

### Summary of Invention

### Technical Problem

The phosphor-integrated particle of which surface is modified with a biological substance-binding protein like antibody or streptavidin (in the present specification, referred to as a "protein-modified phosphor-integrated particle") has a problem that, during the storage till use after manufacture, precipitation or aggregation easily occurs in general. There has been a case in which, in a fluorescent image obtained by immunostaining of a specimen like tissue fragments by using a fluorescent staining liquid that is prepared from a solution containing those precipitates or aggregates, a coarse mass of bright spots that is believed to be caused by an aggregated protein-modified phosphor-integrated particle is generated to prevent the correct measurement of the number of bright spots. In order to avoid such situation, for a case in which a fluorescent staining liquid is prepared by using a protein-modified phosphor-integrated particle after storage for a long period of time, it is necessary to have a pretreatment requiring complex operations like carrying out a filter treatment after having in advance a solvent replacement by repeating a suitable number of re-dispersion of the protein-modified phosphor-integrated particle based on centrifugation, removal of a supernatant, dilution with a solvent for staining, and ultrasonication.

Furthermore, by using a particular storage liquid as described in Patent Literature 3, the dispersion stability during storage of the protein-modified phosphor-integrated particle can be improved to a certain extent. However, there is still room for improvement in enhancement of the dispersion stability during storage of the protein-modified phosphor-integrated particle, and also for various protein-modified phosphor-integrated particles to be developed in future, development of a new means for enhancing the dispersion stability during storage is in need.

An object of one embodiment of the present invention is to provide a means for enhancing the dispersion stability of a protein-modified phosphor-integrated particle during storage.

### Solution to Problem

One embodiment of the present invention relates to a method for manufacturing a purified product of a protein-modified phosphor-integrated particle including a purification step for separating protein-modified phosphor-integrated particles from an impurity by bringing a solution containing the protein-modified phosphor-integrated particles and the impurity originating from the manufacturing process thereof into contact with a filter on which a substance capable of reversibly binding to the biological substance-binding protein (in the present specification, referred to as a "protein-binding substance") is supported.

The biological substance-binding protein is preferably an antibody, and the protein-binding substance is preferably protein A, protein G, or protein L.

The protein-modified phosphor-integrated particle is preferably a fluorescent premix particle in which a phosphor-integrated particle modified with a first reactive substance and a biological substance-binding protein modified with a second reactive substance are conjugated to each other based on an interaction between the first reactive substance and the second reactive substance. The first reactive substance is preferably streptavidin and the second reactive substance is preferably biotin.

The protein-modified phosphor-integrated particle is preferably a particle which has 1000 to 5000 biological substance-binding proteins per phosphor-integrated particle.

The average particle diameter of the phosphor-integrated particle is preferably 30 to 300 nm.

One embodiment of the present invention relates to a method for manufacturing a fluorescent staining liquid containing a purified product of a protein-modified phosphor-integrated particle which is obtained by the above manufacturing method.

One embodiment of the present invention relates to a purified product of a protein-modified phosphor-integrated particle which is obtained by the above manufacturing method. Furthermore, another embodiment of the present invention relates to a fluorescent staining liquid containing the purified product.

Furthermore, one embodiment of the present invention relates to a filter for purification of a protein-modified phosphor-integrated particle provided with a filter which has a pore with size allowing passage of a protein-modified phosphor-integrated particle, and a protein-binding substance supported on the filter.

### Advantageous Effects of Invention

According to one embodiment of the present invention, aggregation or precipitation during storage of protein-modified phosphor-integrated particles can be suppressed, and a purified product of a protein-modified phosphor-integrated particle having high dispersion stability is obtained. By using this purified product of a protein-modified phosphor-integrated particle, a fluorescent staining liquid can be quickly prepared, and a fluorescent image with suppressed coarse mass of bright spots based on an aggregated mass of particles, which become a hurdle for quantification of a biological substance, can be obtained.

### Brief Description of Drawings

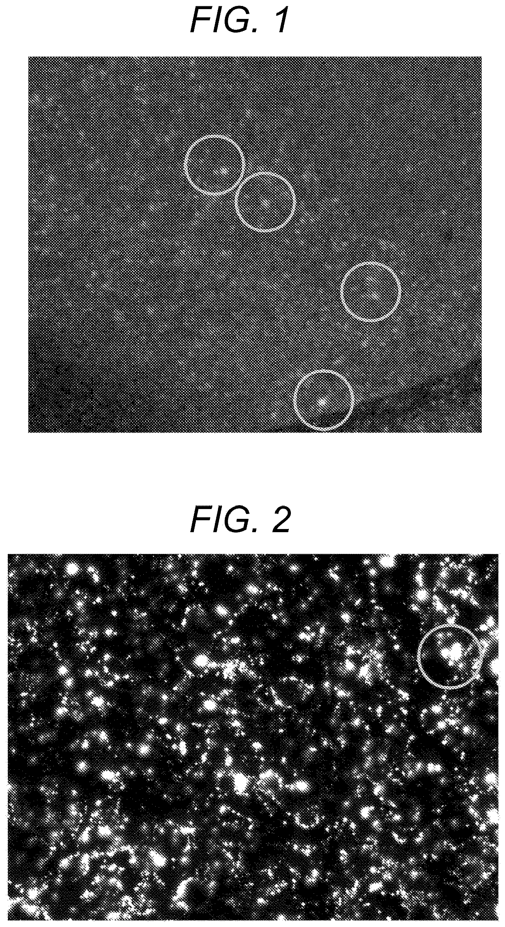

Fig. 1 shows an exemplary image of a dispersion liquid (non-purified product) of a TAMRA-integrated silica particle directly conjugated to an anti HER2 antibody in <Evaluation of dispersion stability> of Examples, in which the image is photographed by using a fluorescent microscope. From the inside of the circle, extremely high-luminance bright spots (aggregated mass) are confirmed.
Fig. 2 shows an image of a stained slide which has been prepared by using a fluorescent staining liquid prepared from the dispersion liquid obtained from Comparative Example 2 in <Evaluation of staining performance> of Examples, in which the image is photographed by using a fluorescent microscope. From the inside of the circle, cluster-shaped bright spots (aggregated mass) are confirmed.

### Description of Embodiments

### - Method for manufacturing purified product of protein-modified phosphor-integrated particle -

The method for manufacturing a purified product of a protein-modified phosphor-integrated particle according to one embodiment of the present invention includes a purification step for separating protein-modified phosphor-integrated particles from an impurity by bringing a solution containing the protein-modified phosphor-integrated particles and impurity originating from the manufacturing process thereof into contact with a filter having a protein-binding substance supported thereon.

Inventors of the present invention found that, as one factor for causing a decrease in the dispersion stability of protein-modified phosphor-integrated particles during storage, various impurities originating from the manufacturing process thereof are mixedly present, and the impurities can be removed at very high level by bringing a solution of protein-modified phosphor-integrated particles into contact with a filter on which a substance capable of reversibly binding to the biological substance-binding protein is supported, and, with a purified product obtained therefrom, the decrease in dispersion stability during the storage can be suppressed.

For manufacturing a protein-modified phosphor-integrated particle, after performing a step of conjugating a biological substance-binding protein to a surface of a phosphor-integrated particle, a step of repeating multiple times centrifuge and dispersion with a pH buffer solution (washing step) is carried out as described in the aforementioned Patent Literature 2 (see, paragraphs 0067, 0134, or the like), and it is believed that the impurities in a solution, in which the particles are contained, are sufficiently removed by this step. However, it was found out by the inventors of the present invention that the impurities that are not entirely removed by this method are one cause of the aggregation or precipitation occurring during storage of the particles, and the aggregation or precipitation occurring during storage of the particles can be suppressed by removing those impurities. It was unexpected even by the inventors of the present invention that the aggregation or precipitation occurring during storage of the particles can be suppressed based on that.

As for the impurities that cannot be sufficiently removed by a conventional washing step, a raw particle material which has not been consumed by particle forming reaction due to secondary interaction or the like between impurities contained in a raw material or a reagent, a surfactant that cannot be removed by washing, a protein not bound to the particles as it becomes non-reactive due to degeneration, or the like, can be mentioned. It is recognized that those impurities exhibit a negative influence on the dispersion stability of a protein-modified phosphor-integrated particle and also on the staining property by fluorescent (immuno) staining.

It was also found out by the inventors of the present invention that the manufacturing method according to one embodiment of the present invention is very effective for enhancing the dispersion stability of particles when a solution containing protein-modified phosphor-integrated particles, which are obtained by reacting in advance (premixing) a streptavidin-modified phosphor-integrated particle with a biotin-labeled secondary antibody described particularly in novel embodiment, specifically, in the embodiment disclosed in the aforementioned Patent Literature 1 (secondary antibody method with combined use of avidin-biotin).

According to this manufacturing method, as the protein-binding substance supported on a filter and the biological substance-binding protein constituting the protein-modified phosphor-integrated particle bind to each other, the protein-modified phosphor-integrated particle is captured by a filter while an impurity not reacting with the protein-binding substance is eluted without being captured by a filter, and thus they can be separated from each other.

In general, the "manufacturing method" of the present invention additionally includes, after the "purification step", a release step for separating the protein-modified phosphor-integrated particle captured by a filter from a filter. The release step can be carried out, for example, by flowing an elution liquid to a filter, or the like.

By collecting protein-modified phosphor-integrated particles that are released from a filter by the release step, a purified product of a protein-modified phosphor-integrated particle can be obtained.

The elution liquid is not particularly limited, but it is preferably a liquid which can sufficiently release the protein-modified phosphor-integrated particle from a filter, and it is also preferably a liquid not causing any loss of a structure or a function of the protein-modified phosphor-integrated particle or constitutional components thereof. Although a suitable liquid can be used depending on the constitutional components of the protein-modified phosphor-integrated particle or type of the protein-binding substance to be contained in a filter or the like, glycine-HCl or the like is suitably used, for example.

Furthermore, after the "purification step" or the "release step", a step for washing a filter or collected protein-modified phosphor-integrated particles, or a necessary treatment among centrifugation treatment, purification treatment using column, and washing treatment that are the same as conventional treatments may be carried out, if necessary.

Furthermore, the protein-binding substance supported on a filter captures not only the protein-modified phosphor-integrated particle but also a non-reacted biological substance-binding protein when the non-reacted biological substance-binding protein is contained in a solution to be contacted with the filter. However, by suitably carrying out the release step, the protein-modified phosphor-integrated particle and non-reacted biological substance-binding protein can be appropriately separated from each other according to the molecular sieve function of a filter.

Specifically, because the protein-modified phosphor-integrated particle generally has a larger size than the biological substance-binding protein and is a particle in which plural biological substance-binding proteins are conjugated per single particle, it has a tendency of being strongly captured by a filter compared to a non-reacted biological substance-binding protein, and thus it is difficult to pass through a filter. As such, when there is a flow of an elution solution, the protein-modified phosphor-integrated particle has slow time for elution from a filter while the non-reacted biological substance-binding protein has fast time for elution from a filter. Due to this reason, according to fractional collection of a fraction containing a large amount of a protein-modified phosphor-integrated particle (late phase of elution), a "purified product" having less content of the non-reacted biological substance-binding protein can be obtained.

Furthermore, on the contrary, when an elution solution with weak elution power is used, the protein-modified phosphor-integrated particle heavier than the biological substance-binding protein is more quickly eluted and released, and thus by fractional collection of a fraction containing a large amount of a protein-modified phosphor-integrated particle (initial phase of elution), a "purified product" having less content of the non-reacted biological substance-binding protein can be obtained.

### <<Purified product of protein-modified phosphor-integrated particle>>

The purified product of a protein-modified phosphor-integrated particle according to one embodiment of the present invention is obtained by a method including the aforementioned purification step.

Furthermore, because the residual amount of an impurity is extremely small in the purified product, the amount of an impurity before carrying out the manufacturing method is also very small, and none of them can be quantified with sufficient accuracy by a known means, it has to be said that, to solve the aforementioned problems, for example, specifying the preferred amount of an impurity in the purified product is impossible or impractical. As such, it is reasonable to specify that the purified product is a product obtained by the aforementioned manufacturing method.

### - Protein-modified phosphor-integrated particle -

The protein-modified phosphor-integrated particle is a phosphor-integrated particle of which surface is modified with a biological substance-binding protein. The method for modifying a phosphor-integrated particle with a biological substance-binding protein can be suitably selected depending on a method for fluorescent labeling of a biological substance as a target for fluorescent staining. As for the protein-modified phosphor-integrated particle, a fluorescent premix particle in which a phosphor-integrated particle modified with a first reactive substance and a biological substance-binding protein modified with a second reactive substance are conjugated to each other based on an interaction between the first reactive substance and the second reactive substance is preferable.

The protein-modified phosphor-integrated particle is preferably a particle in which the number of the conjugated biological substance-binding protein per phosphor-integrated particle is preferably 1000 or higher, and more preferably 1000 to 50000.

By controlling various conditions for manufacturing a protein-modified phosphor-integrated particle, a particle having the aforementioned number of biological substance-binding protein can be obtained.

Number of the conjugated biological substance-binding protein per phosphor-integrated particle can be calculated by the method described in WO 2015/159776 A, for example. Furthermore, in the case of the above fluorescent premix particle, the number can be obtained by the following methods (i) to (iv) in which the method described in WO 2015/159776 A is applied.
(i) Number of a first reactive substance, which has been introduced to a surface of a phosphor-integrated particle, per particle is measured.
(ii) Coefficient showing the maximum number of a second reactive substance (biological substance-binding protein modified with second reactive substance) that can bind to that single first reactive substance is determined (e.g., when the first reactive substance is avidin or the like, the coefficient is 4, and when the first reactive substance is an anti hapten antibody, the coefficient is 1).
(iii) The value obtained by multiplying the number per particle of above (i) by the coefficient of above (ii) is regarded as the maximum value of the number of a biological substance-binding protein that can be conjugated per phosphor-integrated particle.
(iv) When the amount of the biological substance-binding protein modified with a second reactive substance, which has been added to have a reaction, is higher than the maximum value of (iii), the maximum value is regarded as the number of a biological substance-binding protein per phosphor-integrated particle, and, when the amount of the biological substance-binding protein modified with a second reactive substance is smaller than the maximum value of (iii), the number obtained by dividing the total addition number of a biological substance-binding protein modified by a second reactive substance by the number of a phosphor-integrated particle is regarded as the number of a biological substance-binding protein per phosphor-integrated particle.

### <<Biological substance-binding protein>>

The biological substance-binding protein is a protein capable of binding to a biological substance, and it is used for direct or indirect binding of a phosphor-integrated particle to a target biological substance as a subject for fluorescent staining. The biological substance-binding protein can be suitably selected depending on the method for fluorescent labeling of a target biological substance and also a mode of carrying out the modification of a phosphor-integrated particle, but it is preferably an antibody.

When immunostaining is carried out as fluorescent staining, for example, if a phosphor-integrated particle is directly bound to the target biological substance, an antibody (primary antibody) specifically binding to the target biological substance can be employed as a biological substance-binding protein.

On the other hand, for immunostaining, when a phosphor-integrated particle is indirectly conjugated to a target biological substance, (i) an antibody specifically binding to a primary antibody (secondary antibody), (ii) a higher antibody (tertiary antibody) like an antibody specifically binding to a secondary antibody, or (iii) a reactive substance (first reactive substance) specifically binding to a reactive substance (second reactive substance) which modifies a primary antibody or a secondary antibody can be used as a biological substance-binding protein.

Furthermore, the expression "surface of a phosphor-integrated particle is modified with a biological substance-binding protein" includes not only a case in which the biological substance-binding protein is directly bound to a surface of a phosphor-integrated particle but also a case in which the biological substance-binding protein is bound via a certain bond.

For example, in the aforementioned fluorescent premix particle, the biological substance-binding protein binds to the furthest site from the surface of a phosphor-integrated particle.

### <Antibody>

As the biological substance-binding protein, it is preferable to use an antibody (immunoglobulin). The antibody is not particularly limited as long as it is an antibody capable of having direct or indirect binding to a target biological substance in immunostaining. The antibody can be any one of the aforementioned primary antibody and secondary antibody, and a higher antibody like tertiary or higher antibody.

The primary antibody is an antibody which recognizes an epitope unique to an antigen and binds thereto, and, from the viewpoint of the stability of quantification of a target biological substance, a monoclonal antibody is preferable than a polyclonal antibody, and, when two or more kinds of a monoclonal antibody are used as a mixture, combination of monoclonal antibodies showing specific binding to an epitope that is different for each antibody is preferable.

The immunization animal for producing a primary antibody is not particularly limited, and it can be selected from common animals. Examples of the immunization animal include a mouse, a rat, a guinea pig, a rabbit, a sheep, and a goat.

The secondary antibody or a higher antibody is an antibody which recognizes an epitope present in a region (Fc or the like) not involved in binding to an epitope unique to a primary antibody or a lower antibody, and preferably a target biological substance (antigen) of those antibodies, and binds thereto. From the viewpoint of the stability of quantification of a target biological substance, the secondary antibody or the like is preferably a monoclonal antibody. However, from the viewpoint of the economic efficiency, it is also possible to use a polyclonal antibody.

As for the immunization animal for producing a secondary antibody or the like, a suitable animal can be selected from animal species that are exemplified as an immunization animal for producing a primary antibody, depending on the animal species of producing a primary antibody or the like or the animal species forming a region like Fc. For example, when a natural type mouse antibody (IgG produced by a mouse) is used as a primary antibody, it is appropriate to use an antibody which is produced by an immunization animal other than mouse (e.g., rabbit or the like) and specifically binds to the mouse IgG, as a secondary antibody.

Any iso type of the antibody can be used. However, it is generally IgG or IgM, and IgG is particularly preferable. As long as it has an ability of specifically recognizing and binding to a target biological substance or a lower antibody, the antibody may be a natural type antibody like full-length IgG, or a non-natural type antibody like antibody fragment including Fab, Fab', F(ab')₂, Fv, and scFv and artificial antibody multi-functionalized (e.g., to have multivalency or multi-specificity) by using those antibody fragments. Furthermore, the antibody may be a natural type antibody originating from a specific immunization animal (e.g., mouse antibody produced by a mouse), a chimeric antibody which is produced by an artificial means using a vector or the like, a humanized antibody, or a complete human antibody.

When protein A, protein G, or protein L is used as a protein-binding substance, the binding property (affinity) between those proteins and an antibody varies depending on a region, an animal type, a subclass, or the like of an antibody, and thus it is necessary to use a suitable antibody.

For example, from the viewpoint that protein A and protein G mainly recognize and bind to an Fc region, it is preferable to use a full-length antibody or an antibody fragment having an Fc region (since protein G also binds weakly to an Fab region, there may be also a case in which an antibody fragment including an Fab region can be used). Meanwhile, as protein L recognizes and binds to a light chain (κ light chain), it is also possible to use an antibody fragment not containing any Fc region like scFv and Fab. Furthermore, protein A strongly binds to human IgG1, IgG2, and IgG4 but hardly binds to human IgG3, for example. Furthermore, although it strongly binds to mouse IgG2a, IgG2b, and IgG3, the binding to mouse IgG1 becomes weak in a buffer under conditions of living body (Tris-HCl or sodium phosphate buffer), and therefore, depending on an animal type, it is preferable to use a subclass antibody having high binding property.

### <First reactive substance and second reactive substance>

The first reactive substance and the second reactive substance are a combination of substances which specifically bind to each other based on their interaction, and they are all selected from substances which show no cross-reaction with the specific binding between a biological substance-binding protein and a target biological substance to which the biological substance-binding protein binds (target protein, or higher antibody like secondary antibody and tertiary).

Both the first reactive substance and the second reactive substance are not limited, but they can be selected from those used in known staining methods for indirect binding of a target biological substance (antigen or the like) to a labeling substance (phosphor or the like), for example.

For example, based on the immunostaining method using an avidin-biotin complex (ABC method), avidins like avidin, streptavidin, and neutravidin, and preferably, streptavidin can be selected as the first reactive substance and biotin can be selected as the second reactive substance. As four biotins bind to one avidin, this embodiment allows detection of a target biological substance with even higher sensitivity. For manufacturing a fluorescent premix particle, in particular, even more biological substance-binding proteins can be conjugated to one phosphor-integrated particle, and thus this embodiment is particularly preferable.

Furthermore, instead of the combination of avidins and biotin, a hapten substance (not having immunogenicity, but exhibiting antigenicity, and being reactive with an antibody and having a relatively low molecular weight) and an anti hapten antibody (e.g., dikoxigenin and anti dikoxigenin antibody, fluorescein isothiocyanate (FITC) and anti FITC antigen) can be also used as the first reactive substance and the second reactive substance. They can be also used as a biological substance-binding protein in the third and the fourth embodiments of the immunostaining method to be describer later, for example.

### <<Phosphor-integrated particle>>

The phosphor-integrated particle (particle before modification with a biological substance-binding protein) is, although not particularly limited, preferably a nano-sized particle (1 µm or less in diameter) having plural phosphors (e.g., fluorescent pigment) fixed and integrated in the inside or on the surface of a particle to be a base body consisting of an organic material or an inorganic material, and it is preferably a particle which can emit, as a single particle, fluorescence with sufficient luminance.

Compared to a case in which a phosphor is singly used (as a single molecule with no integration), the phosphor-integrated particle shows higher fluorescence intensity (luminance) when labeling a target biological substance and has higher distinguishability from any noise such as auto fluorescence of a cell or other pigments, and also is less likely to have deterioration caused by irradiation with excitation light (higher light resistance) compared to a phosphor itself, and thus it is preferably used for fluorescent staining.

The phosphor to be integrated in a phosphor-integrated particle is not particularly limited, but various types of known organic fluorescent pigment molecules or semiconductor nano particles (may be also referred to as quantum dots) can be used, for example. Hereinbelow, the phosphor-integrated particle in which an organic fluorescent pigment is used as a phosphor is referred to as an organic fluorescent pigment-integrated particle, and the phosphor-integrated particle in which a semiconductor nano particle is used as a phosphor is referred to as an inorganic phosphor-integrated particle.

As for the phosphor used for manufacturing a phosphor-integrated particle, it is preferable to select a phosphor which emits fluorescence with desired wavelength (color) depending on desired use. When there are two or more kinds of a target biological substance as a subject for fluorescent staining, it is preferable to select a combination of phosphors which emit fluorescence with different wavelength corresponding to each substance and manufacture a phosphor-integrated particle in which each phosphor is integrated. When such two or more kinds of a phosphor are used, it is preferable to select phosphors of which light-emitting wavelength peaks are separated from each other by a distance of 100 nm or more.

### (1) Organic fluorescent pigment-integrated particle

The organic fluorescent pigment-integrated particle is preferably a nano-sized fluorescent particle having plural organic fluorescent pigments fixed and integrated in the inside or on the surface of a substance to be a base body of a particle.

Examples of the organic fluorescent pigment include a fluorescein-based pigment, a rhodamine-based pigment, an Alexa Fluor (registered trademark, manufactured by Invitrogen)-based pigment, a BODIPY (registered trademark, manufactured by Invitrogen)-based pigment, a Cascade (registered trademark, Invitrogen)-based pigment, a coumarin-based pigment, an NBD (registered trademark)-based pigment, a pyrene-based pigment, a cyanine-based pigment, a perylene-based pigment, an oxazine-based pigment, a pyrromethene-based pigment, and a fluorescent pigment composed of a low-molecular organic compound (which is not a polymeric organic compound such as a polymer). Among them, sulforhodamine 101 and Texas Red (registered trademark) as a hydrochloride salt of sulforhodamine 101, a perylene-based pigment like perylenediimide, and a pyrromethene-based pigment like pyrromethene 556 have relatively high light resistance, and therefore preferable.

As a base body constituting the organic fluorescent pigment-integrated particle, a substance capable of integrating an organic fluorescent pigment by physical or chemical binding force, for example, a resin, silica, or the like, can be mentioned. Specifically, a resin such as polystyrene, polyamide, polylactic acid, polyacrylonitrile, polyglycidylmethacrylate, polymelamine, polyurea, polybenzoguanamine, polyfuran, polyxylene, phenol resin, or ASA resin (acrylonitrile-styrene-methyl acrylate copolymer); polysaccharides; silica or the like; and substances capable of stably integrating an organic fluorescent pigment can be mentioned. A hydrophobic compound like polystyrene, polymelamine, and silica, in particular, a melamine resin and a styrene resin are preferable as they allow easy manufacture of a fluorescent pigment-integrated particle and also obtainment of a particle with high light emission intensity.

For example, the organic fluorescent pigment-integrated particle manufactured by using a fluorescent pigment like perylenediimide, sulforhodamine 101 or a hydrochloride salt thereof (Texas Red), and pyrromethene as a phosphor and a resin like melamine resin and styrene resin as a base body has excellent labeling performance or the like, and thus preferable as the aforementioned phosphor-integrated particle.

### (2) Inorganic phosphor-integrated particle

The inorganic phosphor-integrated particle is preferably a nano-sized fluorescent particle having plural semiconductor nano particles integrated in the inside or on the surface of a substance to be a base body of a particle.

The semiconductor nano particle is not particularly limited, and examples thereof include a quantum dot containing a compound of Group II-VI, a compound of Group III-V, or an element of Group IV, and a particle dot like CdSe which has been exemplified in WO 2012/133047 A.

Furthermore, a quantum dot in which a semiconductor nano particle is a core and a shell is formed around the core can be also used. Hereinbelow, as a way of describing a semiconductor nano particle having shell, it is described as CdSe/ZnS when the core is CdSe and shell is ZnS.

Specific examples of a semiconductor nano particle having shell include CdSe/ZnS exemplified in WO 2012/133047 A, or the like.

It is also possible that a surface treatment with an organic polymer or the like has been applied to the semiconductor nano particle, if necessary. For example, CdSe/ZnS (manufactured by Invitrogen) of which particle surface is modified with a carboxyl group, CdSe/ZnS (manufactured by Invitrogen) of which particle surface is modified with an amino group, or the like can be used.

As a base body constituting the inorganic phosphor-integrated particle, a substance capable of integrating a semiconductor nano particle by physical or chemical binding force, for example, a resin, silica, or the like, can be mentioned. Examples of the resin include thermosetting resins such as a melamine resin, a urea resin, a benzoguanamine resin, a phenol resin and a xylene resin; and various homopolymers and copolymers produced by use of one or more monomer, such as a styrene resin, a (meth) acrylic resin, polyacrylonitrile, an AS resin (acrylonitrile-styrene copolymer) and an ASA resin (acrylonitrile-styrene-methyl acrylate copolymer).

The organic fluorescent pigment-integrated particle and inorganic phosphor-integrated body are publicly known, and, with regard to details including a phosphor and a base body used for manufacture thereof, manufacturing method thereof, and specific examples of an embodiment, reference can be made to WO 2013/035703 A, WO 2013/147081 A, WO 2014/136776 A, or the like, for example.

### [Average particle diameter of phosphor-integrated particle]

The average particle diameter of a phosphor-integrated particle is preferably 30 to 300 nm, and more preferably 40 to 160 nm. In general, as the particle diameter decreases, higher specific surface area and higher binding force to a specimen are obtained. However, if the average particle diameter is less than 30 nm, there is a case in which bright spots supposed to be observed under fluorescence observation are not observed at all or hardly observed due to a phosphor-integrated particle. On the contrary, if the average particle diameter of a phosphor-integrated particle is more than 300 nm, there may be a case in which bright spots are not separated from each other as too many bright spots are observed or the like, and thus correct counting of bright spots is difficult to achieve.

The variation coefficient representing a deviation in the particle diameter of a phosphor-integrated particle is, although not particularly limited, preferably not more than 20% or so.

The particle diameter of a phosphor-integrated particle can be measured by taking a photographic image using a scanning electron microscope (SEM), measuring the cross-sectional area of a resin particle for fluorescent labeling, followed by assuming a circle having the same area as the measured area, and calculating the diameter of this circle (area equivalent circle diameter). After measuring the particle diameter of each phosphor-integrated particle included in a group with sufficient number (for example, 1000) as described in the above, the average particle diameter is calculated as an arithmetic mean thereof, and the variation coefficient is calculated by the equation: 100 × standard deviation of particle diameter/average particle diameter.

It is possible to have the average particle diameter of a phosphor-integrated particle fallen within a desired range by controlling the manufacturing conditions thereof.

As an exemplary method for manufacturing an organic fluorescent pigment-integrated particle, mention may be made of an emulsion polymerization, in particular, a method in which, while (co)polymerizing monomers for synthesizing a resin (thermoplastic resin or thermosetting resin) to be a base body, a phosphor is added so that the phosphor is introduced to the inside or on the surface of the (co)polymer. In a reaction system of emulsion polymerization, a micelle having aqueous phase as an external side and an oil phase as an internal side is formed by a surfactant, and a state in which the monomer constituting a resin is included in the oil phase at the internal side of micelle is yielded. The polymerization reaction occurs inside the micelle. When an organic fluorescent pigment-integrated particle is synthesized by this emulsion polymerization, by adding 10 to 60% by weight of a surfactant having an emulsifying activity to a raw resin material, a particle with average particle diameter of 30 to 300 nm can be manufactured. Furthermore, if the amount of a surfactant to be used remains constant, the average particle diameter of a fluorescent pigment-integrated particle can be controlled also by modifying the ratio of each of the raw resin material and phosphor used for manufacturing a fluorescent pigment-integrated particle relative to the entire reaction system.

Meanwhile, it is also possible to have the average particle diameter of an inorganic phosphor-integrated particle fallen within a desired range by, after producing an inorganic phosphor-integrated particle, classifying the particle by size selective precipitation method, and collecting a fraction of an inorganic phosphor-integrated particle having pre-determined particle diameter.

The size selective precipitation method is a method in which an adsorbent having a lipophilic group is adsorbed in advance onto a surface of an inorganic phosphor-integrated particle, the inorganic phosphor-integrated particle is dispersed in a lipophilic solvent, and precipitating the particle by adding, in small portions, an amphiphilic additive to the solvent. Because the dispersion property of an inorganic phosphor-integrated particle strongly depends on an interaction between the adsorbing group on a particle surface and a solvent, by slowly adding an additive, an aggregated precipitate is formed from a large-sized inorganic phosphor-integrated particle in order, and, by collecting the precipitate by centrifuge and re-dispersing it in a solvent, an inorganic phosphor-integrated particle with narrow particle diameter distribution can be obtained.

Furthermore, examples of the adsorbent having a lipophilic group include a compound having an alkyl group such as heptane, octane, or dodecane, and a compound with 8 to 12 carbon atoms is preferable.

Furthermore, examples of the lipophilic solvent include pyridine and hexane, and as the amphiphilic additive, chloroform, methanol, or the like are preferably used.

As a lipophilic group capable of adsorbing onto a surface of an inorganic phosphor-integrated particle like quantum dot, a phosphino group such as trioctylphosphine (TOP), a phosphine oxide group such as trioctyl phosphine oxide (TOPOT), a phosphoric acid group, an amino group, or the like can be mentioned.

### <<Method for manufacturing protein-modified phosphor-integrated particle>>

In the first and second embodiments of the immunostaining method to be describer later, a phosphor-integrated particle conjugated with a primary antibody and a secondary antibody as a biological substance-binding protein is used for each, and in the third and fourth embodiments of the immunostaining method, a phosphor-integrated particle conjugated with a first reactive substance (e.g., streptavidin, anti hapten antibody) as a biological substance-binding protein is used as a protein-modified phosphor-integrated particle for both.

For manufacturing a protein-modified phosphor-integrated particle in which the biological substance-binding protein is an antibody (e.g., protein-modified phosphor-integrated particle to be used in the first and second embodiments of the immunostaining method to be describer later), the phosphor-integrated particle is reacted with an antibody. However, for this reaction, to add an excessive amount of an antibody, a non-reacting (not reacting with a particle) antibody may be generally included in a large amount in a solution after the reaction other than the protein-modified phosphor-integrated particle produced therein.

The protein-modified phosphor-integrated particles can be manufactured by a known technique. It is possible that the reactive site contained in the synthesized phosphor-integrated particle and the biological substance-binding protein (primary antibody, secondary antibody, or first reactive substance) are directly conjugated via a covalent bond or the like, or, for indirect conjugation, the phosphor-integrated particle and biological substance-binding protein may be conjugated via a linker specifically by forming a covalent bond based on a reaction between each reactive functional group of a linker and a reactive site contained in the phosphor-integrated particle and a reactive site contained in the biological substance-binding protein by using a linker which has a reactive functional groups at both ends thereof.

Examples of the reactive site included in the biological substance-binding protein include an amino group, a carboxyl group, and a thiol group that are contained in common proteins. The reactive site may be a site originally included in the biological substance-binding protein or a site introduced to the biological substance-binding protein by using a treatment agent (compound) other than linker (e.g., introduced to particle surface according to a reaction with a compound like silane coupling agent). For example, for the introduction of a thiol group to the biological substance-binding protein, a method in which a deprotection treatment is carried out using hydroxylamine after reaction with N-succinimidyl S-acetylthioacetate (SATA) to introduce a thiol group to the amino group originally included in the biological substance-binding protein, or a method in which the disulfide bond (-S-S-) originally included in the biological substance-binding protein is cleaved to generate a thiol group by a treatment using reducing agent like dithiothreitol (DTT) can be mentioned. When such thiol group is utilized as a reactive site included in the biological substance-binding protein, the reactive functional group required to be included in a linker, which is used for binding between the phosphor-integrated particle and biological substance-binding protein, is preferably a functional group capable of reacting with a thiol group, for example, a maleimide group. The linker may have a reactive functional group, which is capable of reacting with the reactive site of the biological substance-binding protein, at one end or both ends thereof, and the reactive functional groups at both ends may be the same or different from each other. The linker may be a compound having in the molecule a chain structure like polyoxyalkylene part, i.e., polyethylene glycol (PEG) chain as a representative example. The function of the linker may be accomplished by the silane coupling agent itself as it is described below.

When a particle having silica as a base body is used as a phosphor-integrated particle, as the reactive site included in the phosphor-integrated particle (silica particle), the silanol group on a silica particle surface may be used, or a reactive site other than the silanol group, which has been introduced to a silica particle surface by using a silane coupling agent, may be used.

Examples of the silane coupling agent used herein include a compound having a hydrolyzable group like alkoxy group, acetoxy group, or halogen atom on one end and a functional group like amino group, mercapto group, or epoxy group on the other hand. Because the hydrolyzable group of a silane coupling agent causes a condensation reaction with a silanol group on silica particle surface (or silanol group of other silane coupling agent) after it turns into a silanol group by hydrolysis, the functional group like an amino group can be introduced to a silica particle surface.

The introduced functional group like amino group itself may be used for a reaction with a reactive site included in the biological substance-binding protein, or it may be used, according to a further reaction with a reactive functional group present on one end of a linker, if necessary, for a reaction between a reactive functional group present on the other end of a linker with a reactive site included in the biological substance-binding protein. For example, when a mercapto group or an amino group introduced by a silane coupling agent is a reactive site included in the phosphor-integrated particle, a linker having a maleimide group or an N-hydroxysuccinimide (NHS) group as a reactive functional group therefor at one end thereof is preferably used.

When a particle having a resin as a base body is used as a phosphor-integrated particle, the reactive site included in the phosphor-integrated particle (resin particle) may be a functional group which is included in a raw material used for synthesizing the resin (monomer or the like) and remains even after the synthesis or a predetermined structure formed by the reaction for synthesizing the resin. It may be also a site introduced, by using a treatment agent (compound) other than aforementioned linker, to the reactive site originally included in the phosphor-integrated particle.

For example, when a use is made of a phosphor-integrated particle having melamine resin as a base body, by reacting a silane coupling agent which has a hydrolyzable group for adsorption to a melamine resin on one end (e.g., trialkoxy group) and an amino group on the other end, or by reacting a linker having an amino group at both ends, an amino group as a reactive functional group corresponding to the reactive site included in the biological substance-binding protein can be introduced to a surface of a melamine resin particle. Furthermore, for the linker having an amino group at both ends, the group responsible for the reaction with one amino group included in the linker is a reactive site included in a melamine resin, for example, a reactive functional group for methylol group (-CH₂OH) contained in methylol melamine, which is generated when melamine and formaldehyde used for synthesis of melamine resin are reacted in advance, or for an ether product (-CH₂OR) which is generated by additional reaction of the methylol group with an alcohol.

Furthermore, when a phosphor-integrated particle having a styrene resin as a base body is used, by using for the styrene synthesis a monomer having a functional group like amino group and epoxy group, which is copolymerizable with styrene, in the side chain, a styrene resin particle having, as a reactive site for having a reaction with a reactive functional group present on one end of a linker, a functional group like amino group and epoxy group on a surface is obtained.

As for the linker and silane coupling agent for the aforementioned reaction, those having various reactive functional groups at both ends are commercially available, and thus can be easily obtained. Furthermore, those having a reactive functional group at both ends can be also synthesized by a known method. For example, as a linker which has an NHS group (reactive functional group capable of reacting with an amino group) at one end of a PEG chain and a maleimide group (reactive functional group capable of reacting with a thiol group) at the other end, a product like "SM(PEG)ₙ" (n = 2, 4, 6, 8, 12, 24) (manufactured by Thermo Fisher Scientific) can be used.

The linker and biological substance-binding protein and/or phosphor-integrated particle can be reacted according to known protocols. Because the modification state of a phosphor-integrated particle by the biological substance-binding protein may vary depending on the reaction conditions between the linker and biological substance-binding protein and/or phosphor-integrated particle, for example, number (density) of the reactive site included in biological substance-binding protein and/or phosphor-integrated particle, ratio of number of molecules (concentration and volume of each solution) between the linker and biological substance-binding protein and/or phosphor-integrated particle to be used for the reaction, type and use amount of a reaction reagent, reaction temperature, reaction time, or the like, it is preferable that they are suitably controlled.

In a step for manufacturing the protein-modified phosphor-integrated particle, it is preferable that the reaction between a linker and the biological substance-binding protein and the reaction between a linker and the phosphor-integrated particle are carried out in turn (or, if possible, simultaneously). For example, it is possible that the phosphor-integrated particle and a linker are first bound to each other, and then, the linker binding to the phosphor-integrated particle at one end thereof and the biological substance-binding protein are bound to each other.

### <Method for manufacturing fluorescent premix particle>

In the fifth and sixth embodiment of immunostaining to be described later, a fluorescent premix particle conjugated to a primary antibody and a secondary antibody, respectively, as a biological substance-binding protein is used as a protein-modified phosphor-integrated particle. This fluorescent premix particle can be produced by conjugating the phosphor-integrated particle modified with a first reactive substance to an antibody modified with second reactive substance by an interaction between the first reactive substance and the second reactive substance. Typically, the fluorescent premix particle can be produced by the following First to Third steps.

### • First step: Step for manufacturing phosphor-integrated particle modified with first reactive substance

First step is a step for manufacturing a phosphor-integrated particle modified with the first reactive substance. This step can be carried out by the same embodiment as the step for manufacturing the phosphor-integrated particle modified with a biological substance-binding protein as explained in <<Method for manufacturing protein-modified phosphor-integrated particle>>

After the reaction of First step, a centrifugal separation treatment, a purification treatment using column, a washing treatment, or the like, which are the same as conventional treatments, are carried out, if necessary. Then, the phosphor-integrated particle modified with a first reactive substance (preferably, streptavidin) is collected and used for Second step. Furthermore, as a preferred embodiment, it is also possible that, to remove immediately the unreacted products or impurity, a purification treatment in which contact with a filter having the protein-binding substance supported thereon is carried out after the reaction of First step.

### • Second step: Step for producing antibody modified with second reactive substance

Second step is a step for producing an antibody modified with the second reactive substance. Typically, by using a linker having a reactive functional group at both ends and reacting the each reactive functional group of a linker with a reactive site included in an antibody and a reactive site included in the second reactive substance to form a covalent bond, the antibody and second reactive substance are conjugated to each other via a linker. For example, when biotin is used as the second reactive substance, this step can be carried out by the same embodiment as the step for producing a biotin-modified antibody, which is used for conventional immunostaining using avidin-biotin complex (ABC method).

As for the linker of Second step, in the same manner as First step, the same linker as the linker explained in <<Method for manufacturing protein-modified phosphor-integrated particle>> can be used. Further, due to the common feature, i.e., the reaction between a linker and a protein, both the reaction mode between the linker and second reactive substance (preferably, biotin) and the reaction mode between the linker and an antibody may be the same as the aforementioned reaction mode between the linker and biological substance-binding protein, or they may be modified, if necessary.

Furthermore, a linker which is previously bound to biotin at one end thereof and has a reactive functional group for the reactive site included in an antibody at the other end thereof (e.g., maleimide group for thiol group) is commercially available as a product like so-called labeling kit including necessary reaction reagents or the like, and customarily used. In case of using such kit, it is sufficient for Second step to have only a reaction for binding a biotinylated linker to an antibody.

In Second step, it is preferable that the reaction between a linker and the second reactive substance (the reaction is not necessary when a product like the aforementioned kit is used) and the reaction between a linker and an antibody is carried out in turn (or, if possible, simultaneously).

After the reaction of Second step, a centrifugal separation treatment, a purification treatment using column, a washing treatment, or the like, which are the same as conventional treatments, are carried out, if necessary, and then, the antibody modified with the second reactive substance is collected and used for Third step. Furthermore, as a preferred embodiment, it is also possible that, after the reaction of Second step, a purification treatment in which contact with a filter having the protein-binding substance supported thereon is carried out may be performed to remove immediately the unreacted products or impurity.

### • Third step: Step for reacting phosphor-integrated particle modified with first reactive substance and antibody modified with second reactive substance

Third step is a step in which the phosphor-integrated particle modified with a first reactive substance, which has been produced in First step, is reacted with the antibody modified with a second reactive substance, which has been produced in Second step, to produce finally a fluorescent premix particle.

Third step may be carried out by mixing and reacting for a predetermined time the phosphor-integrated particle modified with a first reactive substance and an antibody modified with a second reactive substance in a suitable solvent (e.g., buffer solution like PBS).

### <<Solution containing protein-modified phosphor-integrated particle and impurity originating from manufacturing process thereof>>

The solution containing the protein-modified phosphor-integrated particle and impurity originating from the manufacturing process thereof is typically a solution obtained after carrying out a reaction (post-reaction solution) for modifying a surface of the phosphor-integrated particle with the biological substance-binding protein for manufacturing a protein-modified phosphor-integrated particle. The protein-modified phosphor-integrated particle is manufactured by undergoing, as described in the above, a long process like synthesis of a phosphor-integrated particle and a surface modification thereof, and, in the post-reaction solution, unreacted raw materials and impurity like contaminants included in raw materials or reagents are included, together with the protein-modified phosphor-integrated particles that are generated by the reaction.

### <<Filter for purifying protein-modified phosphor-integrated particle>>

The filter for purifying a protein-modified phosphor-integrated particle according to one embodiment of the present invention is provided with a filter, which has a pore with size allowing passage of a protein-modified phosphor-integrated particle, and a protein-binding substance supported on the filter. The filter is preferably used for the above manufacturing method.

The filter before supporting a protein-binding substance is not particularly limited, and a filter made of various materials like porous polymer composed of a crosslinked copolymer can be used. For example, a porous polymer composed of a crosslinked copolymer with dextran or a derivative thereof (allyl dextran or the like) and acrylamide or a derivative thereof (N,N-methylene bisacrylamide or the like) is preferable.

Examples of a commercial product of this filter include (example: product name "Sephacryl S-1000 SF", manufactured by GE Healthcare Japan).

The pore size of the filter is, although not particularly limited, preferably a size which allows passage of a protein-modified phosphor particle that is composed of a phosphor-integrated particle with average particle diameter of 30 to 300 nm.

Depending on a monomer of the crosslinked copolymer (type and blending ratio) or reaction conditions or the like, it is possible to adjust the pore size of a filter.

Furthermore, the filter widely used for antibody purification, e.g., protein A-supported resin "TOYOPEARL AF-rProtein A HC-650F", has an average pore diameter of about 5 nm, and it is not a filter which allows passage of a protein-modified phosphor-integrated particle.

A technique for supporting a protein-binding substance on a filter before having the protein-binding substance supported thereon is not particularly limited. However, it is generally preferable that the crosslinked copolymer of a filter is reacted with a functional group included in each of the protein-binding substance to have a covalent bond.

When a dextran-based copolymer is used as a crosslinked copolymer, for example, according to a treatment of reacting the hydroxyl group included in the copolymer with bromoacetic acid, a carboxymethyl group can be introduced. On the other hand, when protein A, protein G, or protein L is selected as the protein-binding substance, the amino group included in those proteins can be utilized. According to active esterification of the carboxymethyl group with water soluble carbodiimide (WSC) or the like followed by a reaction with the amino group, a covalent bond is formed between those reactive groups so that the protein-binding substance can be supported on a filter.

The filter having the protein-binding substance supported thereon may be filled in the same column as the column for gel filtration used for conventional purification treatment, and used for purification of the protein-modified phosphor-integrated particle.

### <Protein-binding substance>

The protein-binding substance can capture a protein-modified phosphor-integrated particle by having a binding property for the biological substance-binding protein included in a protein-modified phosphor-integrated particle (biological substance-binding protein conjugated to phosphor-integrated particle), and also, by weakening the binding, it can release the protein-modified phosphor-integrated particle (and biological substance-binding protein) (protein-binding substance has a reversible binding property). As for the protein-binding substance, a suitable substance may be selected depending on the biological substance-binding protein.

For example, when purification of a protein-modified phosphor-integrated particle, which has an antibody as biological substance-binding protein, is made, it is preferable to use, as a protein-binding substance, protein A, protein G, or protein L that are known to have a strong reversible binding property for an antibody.

When protein A, protein G, or protein L is used as the protein-binding substance, the binding property (affinity) for those antibodies vary depending on a region, animal species, and subclass of an antibody, and therefore it is necessary to use suitable ones corresponding to the antibody conjugated to a protein-modified phosphor-integrated particle. For example, protein A has a property that it strongly binds to human IgG1, IgG2, and IgG4 but hardly binds to human IgG3, and it strongly binds to mouse IgG2a, IgG2b, IgG3, but binding to mouse IgG1 is only weak in a buffer close to physiological conditions (Tris-HCl of sodium phosphate buffer). Furthermore, for example, as protein A and protein G mainly recognize and bind to an Fc region, they are suitable as a protein-binding substance for purifying the protein-modified phosphor-integrated particle to which a full-length antibody or an antibody fragment having an Fc region is conjugated (because protein G weakly binds also to an Fab region, it may be also used for purification of a protein-modified phosphor-integrated particle to which an antibody fragment containing an Fab fragment is conjugated). On the other hand, as protein L recognizes and binds to a light chain (κ chain), it is preferred as a protein-binding substance for purifying a protein-modified phosphor-integrated particle to which an antibody fragment like scFv and Fab not having an Fc region is conjugated

### - Fluorescent staining liquid -

The fluorescent staining liquid according to one embodiment of the present invention includes a purified product of the protein-modified phosphor-integrated particle, and it is generally a dispersion of the purified product.

In a case in which the purified product of the protein-modified phosphor-integrated particle is a liquid, the fluorescent staining liquid may be the liquid itself, or a liquid in which a purified product of the protein-modified phosphor-integrated particle is dispersed in a suitable dispersion medium. Examples of the dispersion medium include phosphate buffered physiological saline (PBS) containing 1% BSA.

In a case in which the fluorescent staining liquid is used for an embodiment in which two or more types of a target biological substance are taken as a subject for fluorescent labeling, it is possible to contain two or more kinds of a protein-modified phosphor-integrated particle corresponding to each target biological substance. In that case, the two or more kinds of a protein-modified phosphor-integrated particle preferably have fluorescent wavelength peaks that are sufficiently separated from each other so as not to exhibit any negative influence on the recognizabililty of fluorescence (bright spots) of the protein-modified phosphor-integrated particle labeling each target biological substance. For example, they are separated from each other by a distance of 100 nm or more. Furthermore, the fluorescent staining liquid to be used for plural target biological substances as a subject may be a one-liquid type in which two or more kinds of a protein-modified phosphor-integrated particle are included in a single pack (dispersion liquid) or a multi-liquid type in which each protein-modified phosphor-integrated particle is included in a separate pack. Depending on embodiments of the staining method, the fluorescent staining liquid may include, in addition to the one-liquid type or multi-liquid type pack of a protein-modified phosphor-integrated particle, a pack of other reagents (for example, staining liquid for observing cell morphology).

### <<Target biological substance>>

The target biological substance as a subject of the fluorescent staining is preferably at least one biological substance contained in a specimen, and it is particularly preferably a protein. Most preferably, it is a protein (antigen) as a subject of immunostaining which is carried out for quantification or detection mainly in pathological diagnosis. Typically, it is preferably a protein related with pathological diagnosis of cancer (so-called biomarker), for example. Specific examples thereof include a receptor for proliferation factors like Programmed cell death 1 ligand 1 (PD-L1), Cytotoxic T Lymphocyte Antigen-4 (CTLA4), CD8, CD30, CD48, CD59, or Epidermal Growth Factor Receptor (EGFR) (HER1), Human Epidermal Growth Factor Receptor (HER2), HER3, HER4, Vasular Endothelial Growth Factor Receptor (VEGFR), Insulin-like Growth Factor Receptor (IGFR), and Hepatocyte Growth Factor Receptor (HGFR), and, as an inhibitory immune check point molecule present on a surface of T lymphocyte, a protein as a receptor of an immune system like Programmed cell death 1 (PD-1), which is a receptor for the above PD-L1, can be exemplified.

### - Fluorescent staining method -

Although the fluorescent staining liquid can be used for various fluorescent staining methods, typically, it is used for manufacturing, for a tissue slide prepared from tissue fragment as a specimen, a stained glass that is fluorescent-labeled by immunostaining of a target protein contained in a specimen.

A basic embodiment of an analysis like fluorescent staining method using phosphor-integrated particles and pathological diagnosis using prepared stained glass or the like is well known. The fluorescent staining method can be generally carried out by steps like a sample pre-treatment step (e.g., deparaffinization treatment, antigen retrieval treatment, cell fixing treatment, washing treatment, or the like), a staining step (e.g., immunostaining treatment, staining treatment for morphology observation, step for treating phosphor-integrated particles, washing treatment, blocking treatment, or the like), a sample post-treatment step (e.g., sealing treatment, penetration treatment, dehydration treatment, or the like), or the like. Furthermore, the analysis using a complete stained glass can be carried out by observation · image capturing process, image processing · analytical process using a photographed image, or the like. The fluorescent staining method and analysis can be suitably carried out in view of the patent literatures like WO 2013/035688 A, JP 2015-117980 A, WO 2014/136885 A, WO 2016/129444 A, and WO 2015/163209 A, or based on general or publicly known technical features.

The fluorescent staining method carried out by using the aforementioned fluorescent staining liquid is not particularly limited, as long as a predetermined object can be achieved by fluorescent labeling of a target biological substance, but the following first to sixth embodiments can be exemplified as a representative example.

The first embodiment of the fluorescent staining method is a method in which a fluorescent-labeled primary antibody resulting from conjugation between a phosphor-integrated particle and a primary antibody is prepared and a target biological substance (target protein) is directly fluorescent-labeled with the fluorescent-labeled primary antibody to have staining (primary antibody method). In this embodiment, the primary antibody corresponds to a biological substance-binding protein, and the fluorescent-labeled primary antibody corresponds to a protein-modified phosphor-integrated particle.

The second embodiment of the fluorescent staining method is a method in which a primary antibody and a fluorescent-labeled secondary antibody resulting from conjugation between a phosphor-integrated particle and secondary antibody are prepared, a target biological substance (target protein) is reacted with the primary antibody, and then, the primary antibody is reacted with the fluorescent-labeled secondary antibody so that the target protein is indirectly fluorescent-labeled to have staining (secondary antibody method). In this embodiment, the secondary antibody corresponds to a biological substance-binding protein, and the fluorescent-labeled secondary antibody corresponds to a protein-modified phosphor-integrated particle.

The third embodiment of the fluorescent staining method is a method in which a second reactive substance-modified primary antibody resulting from conjugation between a primary antibody and a second reactive substance (e.g., biotin and hapten), and a first reactive substance-modified phosphor-integrated particle resulting from conjugation between a phosphor-integrated particle and a first reactive substance (e.g., avidins, anti hapten antibody) are prepared, a target biological substance (target protein) is reacted with the second reactive substance-modified primary antibody, and then, by utilizing the specific binding of the first reactive substance to the second reactive substance, the first reactive substance-modified phosphor-integrated particle is reacted so that the target protein is indirectly fluorescent-labeled to have staining (primary antibody method with combined use of avidin-biotin and primary antibody method with combined use of hapten-anti hapten antibody). In this embodiment, the first reactive substance corresponds to a biological substance-binding protein, and the first reactive substance-modified phosphor-integrated particle corresponds to a protein-modified phosphor-integrated particle.

The fourth embodiment of the fluorescent staining method is a method in which a primary antibody, a secondary reactive substance-modified secondary antibody resulting from conjugation between a secondary antibody and a second reactive substance, and a first reactive substance-modified phosphor-integrated particle resulting from conjugation between a phosphor-integrated particle and a first reactive substance are prepared, a target biological substance (target protein) is reacted with the primary antibody and subsequently reacted with the second reactive substance-modified secondary antibody, and then, by utilizing the specific binding of the first reactive substance to the second reactive substance, the first reactive substance-modified phosphor-integrated particle is further reacted so that the target protein is indirectly fluorescent-labeled to have staining (secondary antibody method with combined use of avidin-biotin and secondary antibody method with combined use of hapten-anti hapten antibody). Also in this embodiment, the first reactive substance corresponds to a biological substance-binding protein, and the first reactive substance-modified phosphor-integrated particle corresponds to a protein-modified phosphor-integrated particle.

The fifth embodiment of the fluorescent staining method is a modification of the aforementioned third embodiment, and it is a method of using a "fluorescent premix particle" having a primary antibody (primary antibody type fluorescent premix particle method). First, a second reactive substance-modified primary antibody resulting from conjugation between a primary antibody and a second reactive substance, and a first reactive substance-modified phosphor-integrated particle resulting from conjugation between a phosphor-integrated particle and a first reactive substance are prepared followed by in-advance reaction between them, and, based on a reaction between the first reactive substance and second reactive substance, a phosphor-integrated particle conjugated with a primary particle is prepared (premixed). Then, by reacting a target biological substance (target protein) with a fluorescent premix particle, which is a phosphor-integrated particle to which the primary antibody is conjugated by premixing, fluorescent-labeling is carried out to have staining. In this embodiment, the primary antibody corresponds to a biological substance-binding protein, and the fluorescent premix particle corresponds to a protein-modified phosphor-integrated particle.

The sixth embodiment of the fluorescent staining method is a modification of the aforementioned fourth embodiment, and it is a method of using a "fluorescent premix particle" having a secondary antibody (secondary antibody type fluorescent premix particle method). First, a second reactive substance-modified secondary antibody resulting from conjugation between a secondary antibody and a second reactive substance, and a first reactive substance-modified phosphor-integrated particle resulting from conjugation between a phosphor-integrated particle and a first reactive substance are prepared followed by in-advance reaction between them, and, based on a reaction between the first reactive substance and second reactive substance, a phosphor-integrated particle conjugated with a secondary particle is prepared (premixed). Then, by reacting a target biological substance (target protein) with a primary antibody, and then reacting the primary antibody with a fluorescent premix particle, which is a phosphor-integrated particle to which the secondary antibody is conjugated by premixing, fluorescent-labeling is carried out to have staining. In this embodiment, the secondary antibody corresponds to a biological substance-binding protein, and the fluorescent premix particle corresponds to a protein-modified phosphor-integrated particle.

Furthermore, for each embodiment, it is also possible to have a modification such that each of two or more kinds of the target biological substance included in a specimen is fluorescent-labeled with a different kind of a protein-modified phosphor-integrated particle. In that case, just one type of the target biological substance may be fluorescent-labeled in turn, or all target biological substances may be fluorescent-labeled at the same time.

### Examples

Hereinbelow, the present invention is explained in detail in view of Examples, but the present invention is not limited to those Examples.

Summary of Production Examples, Examples, and Comparative Examples are shown in the following table.

**[Table 1]**

| Direct antibody conjugation type (non-premix type) phosphor-integrated particle | Phosphor-integrated particle | | Biological substance-binding protein (antibody) | Purification treatment by column having protein A/G supported thereon |
|---|---|---|---|---|
| | Fluorescent pigment | Base body | | |
| Comparative Example 1 | Red (TAMRA) | Silica | Anti HER2 antibody (primary antibody) | Absent |
| Example 1/1' | Red (TAMRA) | Silica | Anti HER2 antibody (primary antibody) | Present |

| | | | | |
|---|---|---|---|---|
| TAMRA: 5-Carboxytetramethylrhodamine | | | | |

**[Table 2]**

| Antibody conjugation type (premix type) phosphor-integrated particle | Reaction solution 1 | | | | Reaction solution 2 | | | Purification treatment by column having protein A/G supported thereon |
|---|---|---|---|---|---|---|---|---|
| | Phosphor-integrated particle | | First reactive substance | Amount of reaction solution | Biological substance-binding protein (antibody) | Second reactive substance | Amount of reaction solution | |
| | Fluorescent pigment | Base body | | | | | | |
| Comparative Example 2 | Red (TA) | Silica | SA | 25 µL | Anti HER2 antibody (primary antibody) | Biotin | 25 µL | Absent |
| Example 2/2' | Red (TA) | Silica | SA | 25 µL | Anti HER2 antibody (primary antibody) | Biotin | 25 µL | Present |

| | | | | | | | | |
|---|---|---|---|---|---|---|---|---|
| TA: Texas Red SA: Streptavidin | | | | | | | | |

### [Comparative Example 1] Production of TAMRA-integrated silica particle directly conjugated with anti HER2 antibody

According to the following production steps 1-1 to 1-4, a TAMRA-integrated silica particle directly conjugated with anti HER2 antibody was produced.

### [Production step 1-1] Production of TAMRA-integrated silica particle

According to the following production steps (1-1a) to (1-1d), a TAMRA-integrated silica particle in which TAMRA (registered trademark) (5-carboxytetramethylrhodamine), which is an organic fluorescent pigment, is integrated (enclosed) as a phosphor was produced.
Step (1-1a): 2 mg of N-hydrosuccinimide ester of TAMRA (TAMRA-NHS ester) and 400 µL (1.796 mmol) of tetraethoxysilane were admixed with each other.
Step (1-1b): Separate from the above reaction solution, a mixed solution was prepared by mixing 40 mL of ethanol with 10 mL of 14% ammonia water.
Step (1-1c): While stirring the mixed solution prepared in the step (1-1b) at room temperature, the mixed solution prepared in the step (1-1a) was added thereto. For 12 hours after the start of addition, stirring was carried out at room temperature.
Step (1-1d): The reaction mixture was centrifuged for 60 minutes at 10000 G, and the supernatant was removed. After dispersing the precipitates by adding ethanol, the centrifuge was carried out again. Washing with ethanol and purified water was carried out in the same order, once for each.

As a result of performing observation of the obtained TAMRA-integrated silica particle under a scanning type microscope (SEM; model S-800 manufacture by Hitachi), it was found that the average particle diameter of the TAMRA-integrated silica particle is 100 nm, and the variation coefficient of average particle diameter is 15%.

### [Production step 1-2] Introduction of maleimide group to TAMRA-integrated silica particle

According to the following production steps (1-2a) to (1-2g), a maleimide group was introduced to the TAMRA-integrated silica particle which has been obtained from the production step 1-1.
Step (1-2a): 1 mg of the TAMRA-integrated silica particle obtained from the production step 1-1 was dispersed in 5 mL of purified water. Next, 100 µL of aqueous dispersion of 3-aminopropyltriethoxysilane (LS-3150 or KBE-903 manufactured by Shin-Etsu Chemical Co., Ltd.) were added to a dispersion of the particle, and, according to a reaction for 12 hours under stirring at room temperature, an amino group was introduced to a surface of the TAMRA-integrated silica particle.
Step (1-2b): The reaction solution was centrifuged for 60 minutes at 10000 G, and the supernatant was removed.
Step (1-2c): After dispersing the precipitates by adding ethanol, the centrifuge was carried out again. Washing with ethanol and purified water was carried out in the same order, once for each. As a result of carrying out FT-IR measurement of the obtained TAMRA-integrated silica particle modified with amino group, the spectrum originating from the amino group was observed, and the surface modification with an amino group was confirmed.
Step (1-2d): TAMRA-enclosed silica particle modified with amino group, which has been obtained from the step (1-2c), was adjusted to 3 nM by using phosphate buffered physiological saline (PBS) containing 2 mM ethylenediamine tetraacetic acid (EDTA).
Step (1-2e): The liquid after the adjustment of the step (1-2d) was admixed with SM(PEG)₁₂ (manufactured by Thermo Scientific, succinimidyl-[(N-maleimidopropionamid)-dodecaethyleneglycol]ester) so as to have the final concentration of 10 mM, followed by reaction for 1 hour at room temperature.
Step (1-2f): The reaction solution was centrifuged for 60 minutes at 10000 G, and the supernatant was removed.
Step (1-2g): After dispersing the precipitates obtained from the step (1-2f) by adding PBS which contains 2 mM EDTA, the centrifuge was carried out again. Washing was carried out 3 times in the same order. Finally, the precipitates were dispersed again in 500 µL PBS to obtain a dispersion (500 µL) of the TAMRA-integrated silica particle modified with maleimide group.

### [Production step 1-3] Generation of mercapto group in anti HER2 antibody

According to the following steps (1-3a) to (1-3c), a mercapto group-introduced anti HER2 antibody in which a mercapto group (-SH) is generated on an anti HER2 antibody was prepared, and the amount of mercapto group in the antibody was quantified.
Step (1-3a): Anti HER2 antibody (manufactured by Ventana, anti HER2 rabbit monoclonal antibody "4B5", molecular weight: 148000) (100 µg) was dissolve in 100 µL PBS. To the antibody solution, 1 M 2-mercapto ethanol solution (0.002 mL, 0.2 × 10⁻⁵ mol) was added and reacted for 30 minutes at pH 8.5, room temperature. Accordingly, disulfide bond (-S-S-) in the antibody was reduced to generate a mercapto group.
Step (1-3b): The reaction solution after the step (1-3a) was provided to a gel filtration column, and, by removing excess 2-mercaptoethanol, a solution of anti HER2 antibody having a mercapto group generated therein was obtained.
Step (1-3c): 1 µL (1 µg portion) of the antibody having a mercapto group generated therein was fractionated, and, by using Redox Assay Thiol Quantification Kit (product code: TH01D, maker: Metallogenics) which is a kit for quantification of mercapto group, amount of the mercapto group (mole number) was measured. Furthermore, the same volume of the antibody (1 µL) was separately fractionated and subjected to a BCA method to quantify the mass of the fractionated anti HER2 antibody. From the resulting mass and the molecular weight of anti HER2 antibody, mole number of the fractionated antibody was calculated. Furthermore, based on the equation "mole number of mercapto group/mole number of antibody", number of the mercapto group per antibody was calculated, and the result was found to be 1.5.

### [Production step 1-4] Production of TAMRA-integrated silica particle directly conjugated with anti HER2 antibody

According to the following production steps (1-4a) to (1-4c), the TAMRA-integrated silica particle introduced with a maleimide group is conjugated to anti HER2 antibody introduced with a mercapto group to produce a TAMRA-integrated silica particle directly conjugated with anti HER2 antibody.
Step (1-4a): 0.01 µg of the TAMRA-integrated silica particle modified with a maleimide group, which has been obtained from the step (1-2g), and 10 µg of the anti HER2 antibody introduced with a mercapto group, which has been obtained from the step (1-3b), were admixed with each other in 1 mL PBS and reacted for 1 hour at room temperature.
Step (1-4b): By adding 4 µL of 10 mM 2-mercaptoethnaol to the reaction solution after the step (1-4a), the binding reaction was terminated.
Step (1-4c): The liquid obtained from the step (1-4b) was centrifuged for 60 minutes at 10000 G, and the supernatant was removed. After that, the precipitates were dispersed by adding PBS which contains 2 mM EDTA, and the centrifuge was carried out again. Washing was carried out 3 times in the same order. Finally, the precipitates were dispersed again in 500 µL PBS to obtain a dispersion of the TAMRA-integrated silica particle directly conjugated with anti HER2 antibody (directly conjugated by covalent bond).

As a result of carrying out a measurement according to the method described in paragraphs [0135] to [0136] of WO 2016/129444 A, it is believed that, on a surface of one TAMRA-integrated silica particle directly conjugated with anti HER2 antibody, 3000 antibodies on average, or, in terms of the antibody per mm² of the particle surface, 9.555 × 10¹⁶ antibodies are bound.

### [Example 1] Production of purified product of TAMRA-integrated silica particle directly conjugated with anti HER2 antibody by using protein A-bound resin column

After carrying out the production steps 1-1 to 1-4 in the same manner as Comparative Example 1, by using a protein A-bound resin column prepared by the following production step 1-5, a purification treatment based on the following production step 1-6 was carried out.

### [Production step 1-5] Preparation of protein A-bound resin column

By reacting the dextran hydroxyl group of gel permeating carrier "Sephacryl S-1000 SF" (manufactured by GE Healthcare Japan, resin matrix in which allyl dextran and N,N-methylenebisacrylamide are covalently crosslinked) with bromoacetic acid for 16 hours, the carrier surface was carboxymethylated (see, Monchaux, E., and Vermette, P. (2008). Cell adhesion resistance mechanisms using arrays of dextran-derivative layers. J Biomed Mater Res A 85, 1052-1063). In addition, "Sephacryl S-1000 SF" is believed to be a porous body which has a pore allowing infiltration and penetration of a substance with particle diameter of 230 nm (liposome) (see, http://lifesciencedb.jp/dbsearch/Literature/get_pne_cgpdf.php?year=1990&number=3511&file=2Qgovzb50x7RW4I cCUhKPw==).

Subsequently, as water soluble carbodiimide (WSC), a mixture solution containing 1-ethyl-3-(3-dimethylaminopropyl)carbodiimide hydrochloride (EDC: manufactured by Dojindo Laboratories) at a concentration of 400 nM and N-hydroxysuccinic acid imide at a concentration of 100 mM (NHS: manufactured by Thermo Fisher Scientific) was prepared, and, by reacting the mixture solution with the above carboxymethylated carrier, the carboxyl group was converted to an active ester. The resultant was additionally reacted with a solution of protein A (manufactured by Thermo Fisher Scientific, "21184"), and, according to dehydration reaction, the amino group in protein A was bound (immobilized) to the active-esterified carboxyl group. Thus-obtained protein A-bound resin was filled in a column (1 mL syringe tube), and thus a protein A-bound resin column was produced.

### [Production step 1-6] Purification treatment of TAMRA-integrated silica particle directly conjugated with anti HER2 antibody

Through the protein A-bound resin column produced by the production step 1-5, 5 mL of buffer solution A (0.1 M glycine + 1.2 M sodium tartrate, pH 9.0) was flown 3 times to have equilibration. Then, 1.5 mL of a dispersion of TAMRA-integrated silica particle directly conjugated with anti HER2 antibody as obtained by the production step 1-4 was diluted with 1.5 mL buffer solution A and added to the equilibrated protein A-bound resin column so that the anti HER2 antibody (IgG) of the TAMRA-integrated silica particle directly conjugated with anti HER2 antibody was allowed to bind to the protein A of the protein A-bound resin column. After washing the column with 10 mL of buffer solution A, by flowing 3 mL of 0.1 M glycine-HCl (pH 2.8) as an eluent, the TAMRA-integrated silica particle directly conjugated with anti HER2 antibody was desorbed and a purified product was collected.

At that time, impurities not adsorbed onto the resin column (protein A) passed through first, and the TAMRA-integrated silica particle directly conjugated with anti HER2 antibody or non-reacted antibody was collected after flow of the eluent. Furthermore, according to flow of the eluent, non-reacted small antibody is dissociated and eluted due to the molecular sieve function of the resin, and thereafter, the large-sized TAMRA-integrated silica particle directly conjugated with anti HER2 antibody is eluted. The purified product of the TAMRA-integrated silica particle directly conjugated with anti HER2 antibody was collected by obtaining the late-stage fractions of the elution.

### [Example 1'] Production of purified product of TAMRA-integrated silica particle directly conjugated with anti HER2 antibody by using protein G-bound resin column

A purified product of the TAMRA-integrated silica particle directly conjugated with anti HER2 antibody was produced in the same manner as Example 1 except that, instead of the protein A-bound resin column of Example 1, the protein G-bound resin column produced by the following production step 1-7 is used.

### [Production step 1-7] Production of protein G-bound resin column

A protein G-bound resin column was produced in the same manner as the production step 1-5 except that, instead of protein A, protein G (manufactured by Thermo Fisher Scientific, "21193 ") is used.

### [Comparative Example 2] Production of Texas Red-integrated silica particle conjugated with anti HER2 antibody (fluorescent premix particle)

According to the following production steps 2-1 to 2-4, a fluorescent premix particle was produced.

### [Production step 2-1] Production of Texas Red-integrated silica particle

As aphosphor, 3.4 mg of "Texas Red-X" (Sulforhodamine 101-X, manufactured by Sigma Aldrich Company), which is a red organic fluorescent pigment, was admixed with 3 µL of 3-aminopropyltrimethoxysilane (manufactured by Shin-Etsu Silicone, KBM903) in N,N-dimethyl formamide (DMF) to obtain an organoalkoxysilane compound.

The obtained organoalkoxysilane compound (0.6 mL) was admixed with 48 mL of 99% ethanol, 0.6 mL of tetraethoxysilane (TEOS), 2 mL of ultrapure water, and 2.0 mL of 28% by mass ammonia water for 3 hours at 5°C.

The mixture solution prepared in the above step was centrifuged for 20 minutes at 10000 G to remove the supernatant. The precipitates were added with ethanol to disperse them, and rinse was carried out by performing again the centrifuge. Furthermore, by repeating twice the same rinse, Texas Red-integrated silica particle (excitation wavelength: 590 nm, light emission wavelength: 620 nm) was obtained. An SEM observation was carried out for 1000 obtained particles to measure the particle diameter. As a result of calculating the average particle diameter, it was found to be 160 nm.

### [Production step 2-2] Production of Texas Red-integrated silica particle modified with streptavidin

The Texas Red-integrated silica particle obtained from the production step 2-1 was adjusted to a concentration of 3 nM by using PBS in which 2 mM ethylenediamine tetraacetic acid (EDTA) is contained. SM(PEG)₁₂ as a linker was added to the obtained liquid to a final concentration of 10 mM followed by mixing and reaction for 1 hour at 5°C.

The obtained reaction solution was centrifuged for 20 minutes at 10000 G to remove the supernatant. To the resultant, PBS containing 2 mM EDTA was added to disperse the precipitates, and the centrifuge was carried out again at the same conditions. Furthermore, by repeating three times the washing in the same order, Texas Red-integrated silica particle introduced with a maleimide group was obtained.

Meanwhile, 40 µL streptavidin (manufactured by Wako Pure Chemical Industries Ltd.) which has been adjusted to 1 mg/mL was added to 210 µL borate buffer, and, by adding thereto 70 µL of 2-iminothiolane hydrochloride (manufactured by Sigma Aldrich Company) which has been adjusted to 64 mg/mL, they were reacted for 1 hour at room temperature. Accordingly, a mercapto group was introduced to the amino group of streptavidin (-NH-C(=NH²⁺Cl⁻)-CH₂-CH₂-CH₂-SH). The obtained solution was passed through a gel filtration column (Zaba Spin Desalting Columns: manufactured by Funakoshi Co., Ltd.) to obtain streptavidin introduced with mercapto group, which can bind to Texas Red-integrated silica particle introduced with maleimide group.

By using PBS containing 2 mM EDTA, a liquid (740 µL) in which the entire amount (0.04 mg) of the obtained streptavidin introduced with mercapto group and Texas Red-integrated silica particle introduced with maleimide group are admixed with each other to have the silica particle concentration of 0.67 nM was prepared and the reaction was carried out for 1 hour at room temperature. After that, by adding 10 mM mercaptoethanol, the reaction was terminated.

The obtained liquid was concentrated using a centrifuge filter, and, by removing unreacted products using a gel filtration column for purification, Texas Red-integrated silica particle modified with streptavidin was obtained. By using 50 mM Tris solution, dispersion of the Texas Red-integrated silica particle modified with streptavidin of which particle concentration is adjusted to 0.02 nM was prepared (reaction solution 1).

As a result of carrying out a measurement according to the method described in WO 2015/159776 A, it is believed that the obtained Texas Red-integrated silica particle modified with streptavidin is conjugated, on a surface of the Texas Red-integrated silica particle, with streptavidin at density of 0.0311 streptavidins/nm².

### [Production step 2-3] Production of anti HER2 antibody modified with biotin (primary antibody)

As a primary antibody for a target protein HER2, i.e., anti HER2 antibody, Anti Erb 2 antibody [EP1045Y] (manufactured by Abcam plc.), which is a rabbit monoclonal antibody, was used. By dissolving the anti HER2 antibody in 50 mM Tris solution, a primary antibody solution was prepared.

Meanwhile, the linker reagent "Maleimide-PEG₂-biotin" (manufactured by Thermo Fisher Scientific, product number: 21901) was adjusted to 0.4 mM by using DMSO. The obtained linker reagent solution (8.5 µL) was added and admixed with the primary antibody, and, according to a reaction for 30 minutes at 37°C, biotin was allowed to bind to the anti HER2 antibody via PEG chain. The obtained reaction solution was passed through a desalting column to purify the anti HER2 antibody modified with biotin.

Absorbance of the purified primary antibody modified with biotin (anti HER2 antibody modified with biotin) at wavelength of 300 nm was measured by using a spectrophotometer (manufactured by Hitachi, "F-7000"), and thus protein concentration (primary antibody modified with biotin) in the solution was calculated. By using 50 mM Tris solution, concentration of the primary antibody modified with biotin was adjusted to 6 µg/mL so that a solution of primary antibody modified with biotin was obtained (reaction solution 2).

### [Production step 2-4] Production of Texas Red-integrated silica particle conjugated with anti HER2 antibody

The 0.02 nM dispersion (25 µL) of the Texas Red-integrated silica particle modified with streptavidin (reaction solution 1), which has been obtained from the production step 2-2, and 25 µL of the primary antibody solution modified with biotin (concentration of 6 µg/mL) (reaction solution 2), which has been obtained from the production step 2-3, were admixed with each other and reacted for 1 hour at room temperature to produce a dispersion of Texas Red-integrated silica particle conjugated with anti HER2 antibody (fluorescent premix particle).

### [Example 2] Production of purified product of Texas Red-integrated silica particle conjugated with anti HER2 antibody using protein A-bound resin column

After performing the production steps 2-1 to 2-4, a purification treatment based on the following production step 2-5 was carried out by using a protein A-bound resin column, which has been produced in the same manner as the production step 1-5.

### [Production step 2-5] Purification treatment of Texas Red-integrated silica particle conjugated with anti HER2 antibody

Through the protein A-bound resin column, 5 mL of buffer solution A (0.1 M glycine + 1.2 M sodium tartrate, pH 9.0) was flown 3 times to have equilibration. Then, 1.5 mL of a dispersion of Texas Red-integrated silica particle conjugated with anti HER2 antibody as obtained by the production step 2-4 was diluted with 1.5 mL buffer solution A and added to the equilibrated protein A-bound resin column so that the anti HER2 antibody (IgG) of the Texas Red-integrated silica particle conjugated with anti HER2 antibody was allowed to bind to the protein A of the protein A-bound resin column. After washing the column with 10 mL of buffer solution A, by flowing 3 mL of 0.1 M glycine-HCl (pH 2.8) as an eluent, the Texas Red-integrated silica particle conjugated with anti HER2 antibody was desorbed. Then, similar to the production step 1-6, the late-stage fractions of the elution were collected to recover the purified product of Texas Red-integrated silica particle conjugated with anti HER2 antibody.

### [Example 2'] Production of purified product of Texas Red-integrated silica particle conjugated with anti HER2 antibody using protein G-bound resin column

A purified product of Texas Red-integrated silica particle conjugated with anti HER2 antibody was produced in the same manner as Example 2 except that a protein G-bound resin column produced in the same manner as the production step 1-7 is used instead of the protein A-bound resin column of Example 2.

### <Comparison of collection rate>

Collection rate of the protein-modified phosphor-integrated particles resulting from the purification treatment of Examples 1 and 1' and also Example 2 and 2' was measured. The collection rate was calculated from the amount of protein-modified phosphor-integrated particle contained in the dispersion of a protein-modified phosphor-integrated particle before the addition to a column, and the amount of protein-modified phosphor-integrated particle contained in the obtained purified product. The results are shown in Table 3. The collection rate is related to the fluorescence intensity retention rate of a fluorescent staining liquid before and after the purification.

It was found that use of any one of the protein A-bound resin column and protein G-bound resin enables collection of a protein-modified phosphor-integrated particle at a sufficiently high collection rate.

**[Table 3]**

| | | Collection rate (%) |
|---|---|---|
| Example 1 | With purification treatment of direct antibody conjugation type phosphor-integrated particle (protein A) | 51 |
| Example 1' | With purification treatment of direct antibody conjugation type phosphor-integrated particle (protein G) | 64 |
| Example 2 | With purification treatment of premix type phosphor-integrated particle (protein A) | 44 |
| Example 2' | With purification treatment of premix type phosphor-integrated particle (protein G) | 41 |

### <Evaluation of dispersion stability>

The dispersion obtained from Comparative Examples 1 and 2, and also Examples 1 and 2 were diluted with PBS containing 1% BSA such that the protein-modified phosphor-integrated particle has a concentration of 0.002 nM, and thus a fluorescent staining liquid was prepared, which was then stored under dark and refrigeration conditions.

Each fluorescent staining liquid immediately after the preparation (before storage), or after storage for 1 month or 3 months, was subjected to a ultrasonication. The liquid was taken in an amount of 40 µL and placed on an APS-coated glass slide. After that, it was allowed to stand for 2 hours or so at room temperature, and then dried by removing moisture.

With use of a fluorescence microscope "BX53" and a microscope digital camera "DP73" (both manufactured by Olympus Corporation) and an objective lens of ×40, the dried product of the fluorescent staining liquid was irradiated with excitation light. By focusing on the stage height showing bright spots with even luminance, the image was photographed with light exposure time of 500 ms.

For the photographed image of each slide, number of the bright spots showing extremely high luminance (bright spots based on aggregated mass, like bright spots inside the circle in Fig. 1) was counted. The results are shown in Table 4. It was found that the fluorescent staining liquid using the dispersion obtained from Comparative Examples 1 and 2, in which the purification treatment using protein A-supported column has not been carried out, shows the presence of a large amount of residual aggregated mass even after the ultrasonication dispersion treatment, but the fluorescent staining liquid using the dispersion obtained from Examples 1 and 2, in which the purification treatment has been carried out, shows almost no presence of an aggregated mass even after the storage.

**[Table 4]**

| | | Number of bright spots in aggregated mass | | |
|---|---|---|---|---|
| | | Immediately after preparation | After storage for 1 month | After storage for 3 months |
| Comparative Example 1 | Without purification treatment of direct antibody conjugation type phosphor-integrated particle | 4 | 8 | 15 |
| Example 1 | With purification treatment of direct antibody conjugation type phosphor-integrated particle (protein A) | 0 | 0 | 1 |
| Comparative Example 2 | Without purification treatment of premix type phosphor-integrated particle | 20 | 28 | 30 |
| Example 2 | With purification treatment of premix type phosphor-integrated particle (protein A) | 1 | 0 | 1 |

### <Evaluation of staining property>

According to the order shown in the following (1), (2), and (3) a sample pre-treatment step (deparaffinization treatment, retrieval treatment), a staining step (immunostaining treatment), a sample post-treatment step (washing treatment and sealing treatment) were carried out by using the obtained fluorescent staining liquid in the same manner as the evaluation of dispersion stability described in the above. Accordingly, a stained slide based on immunostaining was prepared. After that, by using the prepared stained slide, observation and image capturing were carried out in an order shown in the following (4).

### (1) Sample pre-treatment step

### (1-1) Deparaffinization treatment

A tissue array slide (CB-A712) (HER2 positive staining control sample) manufactured by CosmoBio, for which FISH score has been calculated in advance by using PathVysion HER-2 DNA Probe Kit (manufactured by Abbott Laboratories), was used. The tissue array slide was subjected to a deparaffinization treatment according to the following order (i) to (iii).
(i) In a vessel added with xylene, the tissue array slide was immersed for 30 minutes at room temperature. During the immersion, exchange of xylene was made 3 times.
(ii) In a vessel added with ethanol, the tissue array slide obtained from (i) was immersed for 30 minutes at room temperature. During the immersion, exchange of ethanol was made 3 times.
(iii) In a vessel added with water, the tissue array slide obtained from (ii) was immersed for 30 minutes at room temperature. During the immersion, exchange of water was made 3 times.

### (1-2) Retrieval treatment

The tissue array slide obtained after the deparaffinization treatment was subjected to a retrieval treatment according to the following order (i) to (iv).
(i) Washing for substituting the tissue array slide with water was carried out.
(ii) The tissue array slide obtained from (i) was added to 10 mM citric acid buffer solution (pH 6.0) and subjected to an autoclave treatment at 121°C for 15 minutes.
(iii) The tissue array slide obtained after the autoclave treatment was immersed in a vessel containing PBS for 30 minutes for washing.
(iv) The tissue array slide obtained from (iii) was brought into contact with PBS containing 1% BSA and a blocking treatment was carried out for 1 hour.

### (2) Staining step (immunostaining treatment)

To the tissue array slide obtained from the retrieval treatment (1-2), the fluorescent staining liquid prepared in the above was added dropwise and reacted overnight at 4°C.

### (3) Sample post-treatment step

To the slide obtained after the immunostaining treatment, Entellan New (manufactured by Merck KGaA) was added dropwise at room temperature, and then, after applying a cover glass thereon, the slide was air-dried for 10 minutes at room temperature to carry out a sealing treatment. After that, until the signal measurement, the stained slide obtained after completing the sealing treatment was stored under dark conditions.

### (4) Observation and image capturing

The stained slide obtained after completing the sealing treatment was irradiated with predetermined excitation light (excitation light having wavelength corresponding to the excitation wavelength of TAMRA or Texas Red used as fluorescent pigment) for having fluorescence emission. The stained slide in that state was observed and photographed by using a fluorescence microscope "BX-53" (manufactured by Olympus Corporation) and a microscope digital camera ("DP73", manufactured by Olympus Corporation). The excitation light was set at 545 to 565 nm for TAMRA or 575 to 600 nm for Texas Red according to passage through an optical filter. Furthermore, the fluorescence for observation was set at 570 to 590 nm for TAMRA or 612 to 692 nm for Texas Red according to passage through an optical filter.

Conditions of the excitation light for the microscope observation and image capturing were set such that, for the excitation at 550 nm for TAMRA or at 580 nm for Texas Red, an irradiation energy is 900 W/cm² for each in the vicinity of the center of the visual field. The exposure time for the image capturing was adjusted at 200 milliseconds so as not to cause saturation of the luminance of an image. The bright spots were measured by ImageJ FindMaxims method based on an image taken at 400× magnification.

Presence or absence of cluster-shaped bright spots (aggregated mass) and average luminance per pixel in the photographed image of prepared stained glass are shown in Table 5. Furthermore, the fluorescence stained image obtained by photographing a stained slide, which has been stained by using the fluorescent staining liquid prepared from the dispersion obtained from Comparative Example 2, is shown in Fig. 2.

**[Table 5]**

| | | Image of fluorescent staining | |
|---|---|---|---|
| | | Cluster-shaped bright spots (aggregated mass) | Average luminance per pixel (light exposure; 200 ms) |
| Comparative Example 1 | Without purification treatment of direct antibody conjugation type phosphor-integrated particle | Present | 141 |
| Example 1 | With purification treatment of direct antibody conjugation type phosphor-integrated particle (protein A) | Absent | 105 |
| Comparative Example 2 | Without purification treatment of premix type phosphor-integrated particle | Present | 612 |
| Example 2 | With purification treatment of premix type phosphor-integrated particle (protein A) | Absent | 540 |

## Claims

1. A method for manufacturing a purified product of a protein-modified phosphor-integrated particle, comprising
a purification step for separating protein-modified phosphor-integrated particles from an impurity by bringing a solution containing the protein-modified phosphor-integrated particles and the impurity originating from a manufacturing process thereof into contact with a filter on which a protein-binding substance is supported, wherein
the protein-modified phosphor-integrated particle is a phosphor-integrated particle of which surface is modified with a biological substance-binding protein, and
the protein-binding substance is a substance capable of reversibly binding to the biological substance-binding protein.

2. The manufacturing method according to claim 1, wherein the biological substance-binding protein is an antibody and the protein-binding substance is protein A, protein G, or protein L.

3. The manufacturing method according to claim 1 or 2, wherein the protein-modified phosphor-integrated particle is a fluorescent premix particle, and
the fluorescent premix particle is a particle in which a phosphor-integrated particle modified with a first reactive substance and a biological substance-binding protein modified with a second reactive substance are conjugated to each other based on an interaction between the first reactive substance and the second reactive substance.

4. The manufacturing method according to claim 3, wherein the first reactive substance is streptavidin and the second reactive substance is biotin.

5. The manufacturing method according to any one of claims 1 to 4, wherein the protein-modified phosphor-integrated particle is a particle which has 1000 to 5000 biological substance-binding proteins per phosphor-integrated particle.

6. The manufacturing method according to any one of claims 1 to 5, wherein an average particle diameter of the phosphor-integrated particle is 30 to 300 nm.

7. A method for manufacturing a fluorescent staining liquid containing a purified product of a protein-modified phosphor-integrated particle that is obtained by the manufacturing method according to any one of claims 1 to 6.

8. A purified product of a protein-modified phosphor-integrated particle that is obtained by the manufacturing method according to any one of claims 1 to 6.

9. A fluorescent staining liquid containing the purified product of a protein-modified phosphor-integrated particle according to claim 8.

10. A filter for purifying a protein-modified phosphor-integrated particle, comprising:
a filter, which has a pore with size allowing passage of a protein-modified phosphor-integrated particle; and a protein-binding substance supported on the filter, wherein
the protein-modified phosphor-integrated particle is a phosphor-integrated particle of which surface is modified with a biological substance-binding protein, and
the protein-binding substance is a substance capable of reversibly binding to the biological substance-binding protein.

11. The filter according to claim 10, wherein the filter is a crosslinked copolymer of dextran or a derivative thereof and acrylamide or a derivative thereof.

12. The filter according to claim 10 or 11, wherein the protein-binding substance is protein A, protein G, or protein L.

13. The filter according to any one of claims 10 to 12, wherein an average particle diameter of the phosphor-integrated particle is 30 to 300 nm.
